# EUROPÄISCHE PATENTSCHRIFT

(11) **EP 3 324 927 B1**
(45) Veröffentlichungstag und Bekanntmachung des Hinweises auf die Patenterteilung: **31.07.2019**
(21) Anmeldenummer: 16718665.9
(22) Anmeldetag: 26.04.2016
(51) Int. Cl.: A61K 8/92, A61Q 5/02, A61Q 19/00, A61Q 19/10, A61K 8/46, A61K 8/42, A61K 8/44, A61K 8/86, A61K 8/04

(54) **STABILISIERUNGSGEMISCH**
STABILIZING MIXTURE
MÉLANGE DE STABILISATION

(30) Priorität: 17.07.2015 DE 102015213478
(43) Veröffentlichungstag der Anmeldung: 30.05.2018
(73) Patentinhaber: Henkel AG & Co. KGaA, 40589 Düsseldorf (DE)
(72) Erfinder: SCHRÖDER, Thomas, 22395 Hamburg (DE); HENTRICH, Dirk, 22457 Hamburg (DE)
(86) Internationale Anmeldenummer: PCT/EP2016/059221
(87) Internationale Veröffentlichungsnummer: WO 2017/012727

(56) Entgegenhaltungen:
- EP-A2- 1 977 728
- DE-A1-102006 032 505
- US-A1- 2014 348 927

## Beschreibung

Die vorliegende Erfindung betrifft ein Stabilisierungsgemisch, d.h. eine feinverteilte Dispersion von hydriertem Ricinusöl in Wasser mit anionischen, amphoteren und nichtionischen Tensiden, welches zur Stabilisierung des Perlglanzes von tensidischen Mitteln, insbesondere kosmetischen Mitteln, geeignet ist. Die vorliegende Erfindung betrifft weiterhin ein Kaltverfahren zur Herstellung von tensidhaltigen Mitteln mit Perlglanzeffekt, insbesondere kosmetischen Reinigungsmitteln mit Perlglanzeffekt, sowie die Verwendung des Stabilisierungsgemischs zur Stabilisierung von Perlglanzwachse und/oder Perlglanzpigmente enthaltenden tensidischen Mitteln.

Die Hersteller von kosmetischen Zubereitungen versuchen, durch Perlglanz ihren Produkten ein attraktives, wertvolles und gehaltvolles Erscheinungsbild zu verleihen. Für die moderne Kosmetik werden Perlglanzwachse, insbesondere vom Typ der Glycolmono- und -difettsäureester von Bedeutung, bspw. Stearate, eingesetzt, die bspw. zur Erzeugung von Perlglanz in Haarshampoos und Duschgelen eingesetzt werden. Auch Pigmente wie Titandioxid, Eisenoxide oder Glimmer werden zur Verleihung von Perlglanz in kosmetischen Mitteln verwendet. Im Endprodukt sind diese Perlglanzsubstanzen in einer anionisch basierten Matrix stabilisiert und zwar idealerweise so, dass eine feine und homogene Verteilung der Perlglanzsubstanzen vorliegt und dieser Zustand unter üblichen Lagerungsbedingungen auch beibehalten wird.

Handelsübliche Perlglanzwachse weisen Schmelzpunkte oberhalb von 80 °C auf und lassen sich daher nicht kalt in wässrige Formulierungen einarbeiten. Der Fachmann ist daher bei der Herstellung von tensidischen Mitteln mit Perlglanzeffekt in der Regel gezwungen nach einem Heißverfahren zu arbeiten, d. h. die Wachse aufzuschmelzen und in der Formulierung langsam auskristallisieren zu lassen, wobei die Feinteiligkeit der Kristalle und damit die Brillanz des Perlglanzes eine Funktion der Abkühlgeschwindigkeit ist. Solche Verfahren sind Zeit- und energieaufwendig. Weiterhin ist es bei dieser Herstellungsweise häufig schwierig, reproduzierbare Bedingungen zu gewährleiten, so dass damit häufig Qualitätsschwankungen einhergehen. Verfahren zur Herstellung stabilisierter tensidischer Mittel mit Perlglanzeffekt sollten daher möglichst wenige Schritte mit starkem Wärmeeintrag aufweisen.

Es ist bekannt, hydrierte Ricinusöle zur Stabilisierung von unlöslichen Bestandteilen in tensidischen Mitteln zu verwenden, wobei die hydrierten Ricinusöle in einem Heißverfahren in das tensidische Mittel eingearbeitet werden (DE 102006032505, EP 2037877 B1). Wendet man das hier beschriebene Verfahren an, hat dies aber den gerade beschriebenen Nachteil eines weiteren Herstellungsschritts unter Wärmeeinsatz.

Ein Kaltverfahren zur Einbringung von Perlglanzwachsen in wässrige Tensidlösungen ist aus der DE 19921187 A1 (EP1177274 B1) bekannt. Das Verfahren erfordert aber die gleichzeitige Zugabe der Perlglanzwachse in Kombination mit Polyolestern, welche den Schmelzpunkt der Perlglanzwachse erniedrigen.

Aufgabe der vorliegenden Erfindung war es, eine Zusammensetzung zur Stabilisierung von Perlglanzsubstanzen enthaltenden Mitteln, z. B. Shampoos oder Duschgelen, bereit zu stellen, wobei diese Zusammensetzung mit dem Perlglanzsubstanzen enthaltenden Mittel kalt vermischt werden kann. Eine Aufgabe der vorliegenden Erfindung war es auch, ein einfaches Verfahren zur Stabilisierung von Perlglanzsubstanzen enthaltenden Mitteln bereit zu stellen, bei dem eine weitere Wärmeanwendung nicht erforderlich ist.

Es wurde überraschend gefunden, dass dies durch eine wässrige Zusammensetzung erreicht werden kann, welche hydriertes Ricinusöl enthält und weiterhin eine Kombination von anionischen, nichtionischen und amphoteren Tensiden enthält. In dieser Zusammensetzung liegt durch Verwendung der Tenside in bestimmten Mengen das hydrierte Ricinusöl in sehr fein verteilter Form vor. Überraschend wurde eine deutliche Stabilisierung von Perlglanzsubstanzen enthaltenden tensidischen Mitteln beobachtet, wenn das erfindungsgemäße Stabilisierungsgemisch kalt in ein Perlglanzsubstanzen enthaltendes tensidisches Mittel eingearbeitet wurde.

Die vorliegende Erfindung betrifft:
1. Eine wässrige Dispersion von hydriertem Ricinusöl zur Stabilisierung von Perlglanzwachse und/oder Perlglanzpigmente enthaltenden, wässrigen tensidischen Mitteln, welche, bezogen auf ihr Gesamtgewicht, enthält:
   (a) 1 bis 25 Gew.-% hydriertes Ricinusöl,
   (b) 2 bis 30 Gew.-% eines oder mehrerer anionischer Tenside,
   (c) 2 bis 30 Gew.-% eines oder mehrerer amphoterer Tenside, und
   (d) 2 bis 30 Gew.-% eines oder mehrerer nichtionischer Tenside.
2. Dispersion nach Punkt 1, welche, bezogen auf ihr Gesamtgewicht, enthält:
   (a) 2,5 bis 15 Gew.-% hydriertes Ricinusöl,
   (b) 2,5 bis 10 Gew.-% eines oder mehrerer anionischer Tenside,
   (c) 5 bis 15 Gew.-% eines oder mehrerer amphoterer Tenside, und
   (d) 5 bis 15 Gew.-% eines oder mehrerer nichtionischer Tenside.
3. Dispersion nach Punkt 1 oder 2, wobei die Dispersion kein kationisches Tensid enthält.
4. Dispersion nach Punkt 1 oder 2, wobei die Dispersion keinen Polyolester enthält.
5. Dispersion nach einem der vorhergehenden Punkte,
   wobei als anionisches Tensid (b) ein Alkali- oder Ammoniumsalz des Laurylethersulfats mit einem Ethoxylierungsgrad von 2 bis 4 Ethylenoxidgruppen oder ein Gemisch davon enthalten ist.
6. Dispersion nach einem der vorhergehenden Punkte,
   wobei als das amphotere Tensid (c) ein oder mehrere Betaine enthalten sind, bevorzugt Cocamidopropyl Hydroxysultaine (INCI) und/oder Cocamidopropyl Betaine (INCI).
7. Dispersion nach einem der vorhergehenden Punkte,
   wobei das nichtionische Tensid (d) aus
   - Ethylenoxid-Anlagerungsprodukten an gesättigte lineare Fettalkohole und/oder Fettsäuren mit jeweils 2 bis 30 Mol Ethylenoxid pro Mol Fettalkohol und/oder Fettsäure,
   - C₁₂-C₃₀-Fettsäureestern von Anlagerungsprodukten von 1 bis 30 Mol Ethylenoxid an Glycerin,
   - Anlagerungsprodukten von 5 bis 60 Mol Ethylenoxid an gehärtetes Ricinusöl und
   - Gemischen davon
      ausgewählt ist.
8. Dispersion nach einem der vorhergehenden Punkte, wobei das nichtionische Tensid (d) aus
   - Ethylenoxid-Anlagerungsprodukten an gesättigte lineare Fettalkohole und/oder Fettsäuren mit jeweils 2 bis 30 Mol Ethylenoxid pro Mol Fettalkohol und/oder Fettsäure, und/oder;
   - C₁₂-C₃₀-Fettsäureester von Anlagerungsprodukten von 1 bis 30 Mol Ethylenoxid an Glycerin, und/oder
   - Anlagerungsprodukten von 5 bis 60 Mol Ethylenoxid an gehärtetes Ricinusöl
      ausgewählt ist.
9. Dispersion nach einem der vorhergehenden Punkte, wobei das nichtionische Tensid (d) aus Laureth-4 (INCI), PEG-7 Glycerol Cocoate (INCI), PEG-40 Hydrogenated Castor Oil (INCI) und Gemischen davon ausgewählt ist.
10. Dispersion nach einem der vorhergehenden Punkte, wobei die Dispersion einen pH-Wert im Bereich von 4,5 bis 5,5 aufweist.
11. Dispersion nach einem der vorhergehenden Punkte, wobei die Dispersion, bezogen auf ihr Gesamtgewicht, 40 bis 90 Gew.-%, vorzugsweise 50 bis 85 Gew.-% und insbesondere 60 bis 80 Gew.-% Wasser enthält.
12. Dispersion nach einem der vorhergehenden Punkte, wobei die Dispersion als Träger nur Wasser enthält.
13. Verfahren zur Herstellung eines stabilisierten tensidhaltigen wässrigen Mittels mit Perlglanzeffekt, bei dem eine Dispersion nach einem der Punkte 1 bis 12 in einem Kaltverfahren in ein tensidhaltiges wässriges Mittel, welches Perlglanzwachse und/oder Perlglanzpigmente enthält, eingebracht wird.
14. Verfahren zur Herstellung eines stabilisierten tensidhaltigen wässrigen Mittels mit Perlglanzeffekt, bei dem eine Dispersion nach einem der Punkte 1 bis 12 in einem Kaltverfahren in ein tensidhaltiges wässriges Mittel, welches Antischuppenpigmente, vorzugsweise Zink Pyrithion, enthält, eingebracht wird.
15. Verfahren nach Punkt 13 oder 14, wobei das tensidhaltige wässrige Mittel ein kosmetisches Reinigungsmittel ist, insbesondere ein Shampoo oder Duschgel.
16. Verfahren nach Punkt 13 oder 14, wobei das tensidhaltige wässrige Mittel als Perlglanzpigmente eines oder mehrere aus der Gruppe Titandioxid, Eisenoxide und Glimmer enthält.
17. Verfahren nach einem der Punkte 13 bis16, wobei die Menge des eingebrachten hydrierten Ricinusöls in dem stabilisierten tensidhaltigen wässrigen Mittel 0,1 bis 1 Gew.-% beträgt, bevorzugt 0,2 bis 0,5 Gew.-%.
18. Verwendung einer Dispersion nach einem der Punkte 1 bis 12 zur Stabilisierung von tensidhaltigen, wässrigen Mitteln, insbesondere Shampoos oder Duschgelen, welche Perlglanzwachse und/oder Perlglanzpigmente enthalten.
19. Verwendung einer Dispersion nach einem der Punkte 1 bis 12 zur Stabilisierung von tensidhaltigen, wässrigen Mitteln, insbesondere Shampoos oder Duschgelen, welche Antischuppenpigmente, insbesondere Zink Pyrithion enthalten.
20. Verwendung nach einem der Ansprüche 18 oder 19, wobei der Gewichtsanteil der Dispersion nach einem der Punkte 1 bis 12 am Gesamtgewicht des tensidhaltigen, wässrigen Mittels 0,5 bis 10 Gew.-%, vorzugsweise 1 bis 8 Gew.-% und insbesondere 1,5 bis 6 Gew.-% beträgt.

Bei dem erfindungsgemäßen Stabilisierungsgemisch handelt es sich um eine Dispersion von hydriertem Ricinusöl in einer wässrigen Phase. Durch die verwendeten Tenside und die Einhaltung der angegebenen Mengen liegt das hydrierte Ricinusöl in sehr fein verteilter Form in der wässrigen Phase vor. Die erfindungsgemäße Dispersion wird im Folgenden oft einfach als Stabilisierungsgemisch bezeichnet.

Wird das erfindungsgemäße Stabilisierungsgemisch mit einem wässrigen tensidischen Mittel kalt vermischt, welches Perlglanzsubstanzen wie Perlglanzwachse und/oder Perlglanzpigmente enthält, wird überraschend eine deutliche Stabilisierung der Perlglanzsubstanzen in dem tensidischen Mittel erzielt. Mit Stabilisierung ist erfindungsgemäß gemeint, dass in dem erhaltenen tensidischen Mittel bei üblichen Lagerstabilitätstests, bspw. bei etwa 40 bis 50 °C, im Wesentlichen keine Phasentrennungen auftreten und bei Raumtemperatur über lange Zeiträume keine sichtbaren Veränderungen auftreten.

Unter den wässrigen tensidischen Mitteln, die durch das erfindungsgemäße Stabilisierungsgemisch stabilisiert werden können, sind insbesondere tensidische Reinigungsmittel mit Perlglanz zu verstehen, vorzugsweise kosmetische Reinigungsmittel wie Shampoos oder Duschgele, aber auch beispielsweise Geschirrspülmittel.

Wesentlicher Bestandteil des erfindungsgemäßen Stabilisierungsgemischs ist hydriertes Ricinusöl (INCI: Hydrogenated Castor Oil). Hydriertes Ricinusöl ist im Handel bspw. unter der Bezeichnung Cutina® HR (BASF) erhältlich. Hydrierte Ricinusöle, die in der vorliegenden Erfindung verwendet werden können, weisen üblicherweise einen Schmelzpunkt im Bereich von 60 bis 100 °C auf, bevorzugt etwa 70 bis 90°C, noch bevorzugter etwa 80 bis 90°C, besonders bevorzugt etwa 85 bis 88°C.

Polyalkoxylierte bzw. polyethoxylierte Derivate oder Anlagerungsprodukte von hydriertem Ricinusöl sind erfindungsgemäß nicht von dem Begriff "hydriertes Ricinusöl" umfasst.

Das hydrierte Ricinusöl ist in dem erfindungsgemäßen Stabilisierungsgemisch in einer Menge von 1 bis 25 Gew.-% enthalten, bevorzugt 2,5 bis 15 Gew.-%, weiter bevorzugt 5 bis 10 Gew.-%, jeweils bezogen auf das Gesamtgewicht des Stabilisierungsgemischs.

Weiter enthält das erfindungsgemäße Stabilisierungsgemisch anionische, amphotere und nichtionische Tenside als notwendige Bestandteile. Die Tenside sollten für den Kontakt mit dem menschlichen Körper geeignet sein, insbesondere wenn sie in Endprodukten zur Pflege und/oder Reinigung des menschlichen Körpers zur Anwendung kommen.

Als anionische Tenside sind erfindungsgemäß folgende anionischen oberflächenaktiven Stoffe geeignet. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäss DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 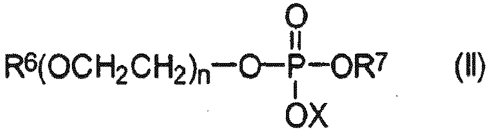 in der R⁶ bevorzugt für einen aliphatischen Kohlenwasserstoffrest mit 8 bis 30 Kohlenstoffatomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁ bis C₄-Kohlenwasserstoffrest, steht,
- sulfatierte Fettsäurealkylenglykolester der Formel (III),

   R¹²CO(AlkO)ₙSO₃M (III)

   in der R¹²CO- für einen linearen oder verzweigten, aliphatischen, gesättigten und/oder ungesättigten Acylrest mit 6 bis 22 C-Atomen, Alk für CH₂CH₂, CHCH3CH2 und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV), wie sie beispielsweise in der EP-B1 0 561 825, der EP-B1 0 561 999, der DE-A1 42 04 700 oder von A. K. Biswas et al. in J. Am. Oil. Chem. Soc. 37, 171 (1960) und F. U. Ahmed in J. Am. Oil. Chem. Soc. 67, 8 (1990) beschrieben worden sind, 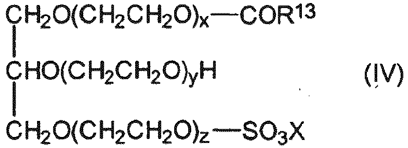 in der R¹³CO für einen linearen oder verzweigten Acylrest mit 6 bis 22 Kohlenstoffatomen steht, x, y und z in Summe für 0 oder für Zahlen von 1 bis 30 stehen, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (IV) eingesetzt, in der R¹³CO für einen linearen Acylrest mit 8 bis 18 Kohlenstoffatomen steht.

Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder Ammoniumsalze des Laurylethersulfates mit einem Ethoxylierungsgrad von 2 bis 4 EO, bspw. das im Handel erhältliche anionische Tensid mit der INCI-Bezeichnung Sodium Laureth Sulfate.

Das anionische Tensid ist in dem erfindungsgemäßen Stabilisierungsgemisch, bezogen auf das Gesamtgewicht des Stabilisierungsgemischs, in einer Menge von 2 bis 30 Gew.-% enthalten, bevorzugt 2 bis 10 Gew.-%, weiter bevorzugt von 2,5 bis 5 Gew.-%.

Als amphotere oder zwitterionische Tenside werden solche oberflächenaktiven Verbindungen bezeichnet, die im Molekül mindestens eine quartäre Ammoniumgruppe und mindestens eine - COO⁽⁻⁾- oder -SO₃⁽⁻⁾- Gruppe tragen. Besonders geeignete amphotere Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat, sowie Sultaine bzw. Sulfobetaine. Besonders bevorzugte amphotere Tenside hierunter sind die unter den INCI-Bezeichnungen Cocamidopropyl Betaine und Cocamidopropyl Hydroxysultaine bekannten Verbindungen.

Unter amphoteren Tensiden werden erfindungsgemäß auch solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Bevorzugte amphotere Tenside hierunter sind das N-Kokosalkylaminopropionat, das Kokosacylaminoethylaminopropionat und das C₁₂-C₁₈-Acylsarcosin.

Das amphotere Tensid ist in dem erfindungsgemäßen Stabilisierungsgemisch, bezogen auf das Gesamtgewicht des Stabilisierungsgemischs, in einer Menge von 2 bis 30 Gew.-% enthalten, bevorzugt 3 bis 20 Gew.-%, weiter bevorzugt von 5 bis 15 Gew.-%.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS (BASF), Dehydol® LT (BASF) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 (BASF) im Handel erhältlich ist (INCI: PEG-40 Hydrogented Castor Oil),
- Polyolfettsäureester, wie beispielsweise das Handelsprodukt Hydagen® HSP (BASF) oder Sovermol-Typen (BASF),
- alkoxylierte Triglyceride,
- alkoxylierte Fettsäurealkylester der Formel (V)

   R¹⁴CO-(OCH₂CHR¹⁵)_{w}OR¹⁶ (V)

   in der R¹⁴CO für einen linearen oder verzweigten, gesättigten und/oder ungesättigten Acylrest mit 6 bis 22 Kohlenstoffatomen, R¹⁵ für Wasserstoff oder Methyl, R¹⁶ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 197 38 866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)x wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Bevorzugt sind solche Alkylpolyglykoside, bei denen R
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen oder
- im Wesentlichen aus C₁₆- bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Weiter bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bevorzugte ethoxylierte Fettalkohole mit einem durchschnittlichen Ethoxylierungsgrad von 2 bis 29 sind beispielsweise Laureth-2, Oleth-2, Ceteareth-2, Laneth-2, Laureth-3, Oleth-3, Ceteareth-3, Laureth-4, Oleth-4, Ceteareth-4, Laneth-4, Laureth-5, Oleth-5, Ceteareth-5, Laneth-5, Deceth-7, Laureth-7, Oleth-7, Coceth-7, Ceteth-7, Ceteareth-7, C11-15 Pareth-7, Laureth-9, Oleth-9, Ceteareth-9, Laureth-10, Oleth-10, Beheneth-10, Ceteareth-10, Laureth-12, Ceteareth-12, Trideceth-12, Ceteth-15, Laneth-15, Ceteareth-15, Laneth-16, Ceteth-16, Oleth-16, Steareth-16, Oleth-20, Ceteth-20, Ceteareth-20, Laneth-20, Steareth-21, Ceteareth-23, Ceteareth-25, Ceteareth-27. Besonders bevorzugt ist der Einsatz eines Gemisches von Steareth-2 und Steareth-21.

Bevorzugt sind weiterhin Mono,- Di- und Tricarbonsäureestern von gesättigten und/oder ungesättigten linearen und/oder verzweigten Carbonsäuren mit Glycerin, welche mit 1 bis 10, insbesondere 7 bis 9 Ethylenoxid-Einheiten ethoxyliert sein können, z. B. PEG-7 Glyceryl Cocoate.

Weitere bevorzugte nichtionische Tenside sind die PEG-Derivate von hydriertem Ricinusöl, die beispielsweise unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil.

Unter oben genannten nichtionischen Tensiden sind die Verbindungen mit den INCI-Bezeichnungen Laureth-4 (INCI), PEG-7 Glycerol Cocoate (INCI), PEG-40 Hydrogenated Castor Oil (INCI) und Gemische davon besonders bevorzugt.

Das nichtionische Tensid ist in dem erfindungsgemäßen Stabilisierungsgemisch, bezogen auf das Gesamtgewicht des Stabilisierungsgemischs, in einer Menge von 2 bis 30 Gew.-% enthalten, bevorzugt 3 bis 20 Gew.-%, weiter bevorzugt von 5 bis 15 Gew.-%.

Das erfindungsgemäße Stabilisierungsgemisch kann zusätzlich ein kationisches Tensid enthalten. Bevorzugt enthält das erfindungsgemäße Stabilisierungsgemisch jedoch kein kationisches Tensid. Beispiele für kationische Tenside sind quartäre Ammoniumverbindungen, Esterquats oder Amidoamine.

Die Gesamtmenge an Tensiden in dem erfindungsgemäßen Stabilisierungsgemisch beträgt bevorzugt 10 bis 40 Gew.-%, bevorzugter 12,5 bis 35 Gew.-% und noch bevorzugter 15 bis 30 Gew.-%, jeweils bezogen auf das Gesamtgewicht des Stabilisierungsgemischs.

Das erfindungsgemäße Stabilisierungsgemisch ist eine wässrige Dispersion und enthält somit als Träger Wasser. Bevorzugte Dispersionen enthalten, bezogen auf ihr Gesamtgewicht, 40 bis 90 Gew.-%, vorzugsweise 50 bis 85 Gew.-% und insbesondere 60 bis 80 Gew.-% Wasser. Bevorzugt ist außer Wasser kein weiterer Träger enthalten.

Das erfindungsgemäße Stabilisierungsgemisch ist üblicherweise sauer und kann als pH-Regulator übliche pH-Regulatoren enthalten, bspw. Zitronensäure. Der pH-Wert des erfindungsgemäßen Stabilisierungsgemischs ist bevorzugt in einem Bereich von 4 bis 6, weiter bevorzugt 4,5 bis 5,5.

Das erfindungsgemäße Stabilisierungsgemisch enthält neben den vorgenannten Tensiden, dem Wasser und dem optionalen pH-Regulator, vorzugsweise weniger als 10 Gew.-%, bevorzugt weniger als 5 Gew.-% und insbesondere weniger als 2 Gew.-% weiterer Bestandteile.

Ein weiterer bevorzugter Gegenstand der vorliegenden Anmeldung ist daher eine wässrige Dispersion von hydriertem Ricinusöl zur Stabilisierung von Perlglanzwachse und/oder Perlglanzpigmente enthaltenden, wässrigen tensidischen Mitteln, welche, bezogen auf ihr Gesamtgewicht, enthält:
(a) 1 bis 25 Gew.-% hydriertes Ricinusöl,
(b) 2 bis 30 Gew.-% eines oder mehrerer anionischer Tenside,
(c) 2 bis 30 Gew.-% eines oder mehrerer amphoterer Tenside, und
(d) 2 bis 30 Gew.-% eines oder mehrerer nichtionischer Tenside
(e) 40 bis 90 Gew.-% Wasser
sowie weniger als 10 Gew.-%, bevorzugt weniger als 5 Gew.-% und insbesondere weniger als 2 Gew.-% weiterer Bestandteile.

Ein besonders bevorzugter Gegenstand der vorliegenden Anmeldung ist eine wässrige Dispersion von hydriertem Ricinusöl zur Stabilisierung von Perlglanzwachse und/oder Perlglanzpigmente enthaltenden, wässrigen tensidischen Mitteln, welche, bezogen auf ihr Gesamtgewicht, enthält:
(a) 2,5 bis 15 Gew.-% hydriertes Ricinusöl,
(b) 2,5 bis 10 Gew.-% eines oder mehrerer anionischer Tenside,
(c) 5 bis 15 Gew.-% eines oder mehrerer amphoterer Tenside, und
(d) 5 bis 15 Gew.-% eines oder mehrerer nichtionischer Tenside
(e) 60 bis 80 Gew.-% Wasser
sowie weniger als 10 Gew.-%, bevorzugt weniger als 5 Gew.-% und insbesondere weniger als 2 Gew.-% weiterer Bestandteile.

Die Viskosität des erfindungsgemäßen Stabilisierungsgemischs ist in einem Bereich von 10000 mPas oder darunter (Brookfield-Viskosimeter, 20°C, 60 s, Spindel 5, 20 UpM).

Das erfindungsgemäße Stabilisierungsgemisch kann weiterhin übliche Zusatzstoffe wie Konservierungsmittel, Parfüms etc. enthalten. Bevorzugte Ausführungsformen sind aber solche, die neben dem hydrierten Ricinusöl, Wasser den anionischen, nichtionischen und amphoteren Tensiden, ggf. einem pH-Regulator und ggf. einem Konservierungsmittel keine weiteren Bestandteile enthalten.

Das erfindungsgemäße Stabilisierungsgemisch kann durch an sich bekannte Verfahren hergestellt werden. Beispielsweise kann ein erstes Gemisch aus einem Teil des Wassers, dem anionischen und dem amphoteren Tensid, sowie ggf. einer schwachen Säure zur pH-Einstellung und einem Konservierungsmittel, vorgelegt und erwärmt werden. Anschließend kann ein zweites Gemisch aus hydriertem Ricinusöl und dem nichtionischen Tensid, das zunächst bei z.B. 90-100 °C aufgeschmolzen worden war, zu dem ersten Gemisch gegeben werden und bei etwa 80 bis 90 °C homogenisiert werden. Schließlich kann der restliche Teil des Wassers im nicht erwärmten Zustand zugegeben werden und das Gemisch wird unter Rühren und Homogenisieren abgekühlt. Soll das Gemisch gleich weiter zur Herstellung eines stabilisierten tensidhaltigen wässrigen Mittels mit Perlglanzeffekt verwendet werden, reicht es, die Abkühlung bis auf eine Temperatur von etwa 30 bis 40°C durchzuführen.

Die vorliegende Erfindung betrifft auch ein Verfahren zur Herstellung eines tensidhaltigen wässrigen Mittels mit Perlglanzeffekt, bei dem ein erfindungsgemäßes Stabilisierungsgemisch in einem Kaltverfahren in ein tensidhaltiges wässriges Mittel, welches Perlglanzwachse und/oder Perlglanzpigmente enthält, eingebracht wird. Das tensidhaltige wässrige Mittel ist erfindungsgemäß bevorzugt ein kosmetisches Reinigungsmittel ist, insbesondere ein Shampoo oder Duschgel, kann aber auch ein nichtkosmetisches Reinigungsmittel sein, z. B. ein Geschirrspülmittel.

Als Perlglanzsubstanzen sind erfindungsgemäß Perlglanzwachse und/oder Perlglanzpigmente vorgesehen. Perlglanzwachse und Perlglanzpigmente sind als solche bekannt. Als Perlglanzwachse kommen beispielsweise in Frage: Alkylenglycolester, Fettsäurealkanolamide, Partialglyceride, Ester von mehrwertigen, gegebenenfalls hydroxysubstituierten Carbonsäuren, Fettalkohole, Fettsäuren, Fettketone, Fettaldehyde, Fettether, Fettcarbonate, Ringöffnungsprodukte von Olefinepoxiden sowie deren Mischungen.

Hydriertes Ricinusöl als Komponente (a) des erfindungsgemäßen Stabilisierungsgemischs gehört erfindungsgemäß aber nicht zu den verwendbaren Perlglanzwachsen.

Perlglanzpigmente sind üblicherweise plättchenförmige Pigmente, welche durch Lichtreflexion eine Glanzwirkung erzielen. Bevorzugte Beispiele sind Glimmer, Titandioxid, Eisenoxide, wie Fe₂O₃ und Fe₃O₄, sowie Kombinationen davon.

Überraschend wurde festgestellt, dass sich erfindungsgemäße Verfahren ebenfalls zur Herstellung eines stabilisierten tensidhaltigen wässrigen Mittels mit Antischuppeneffekt eignet, bei dem ein erfindungsgemäßes Stabilisierungsgemisch in einem Kaltverfahren in ein tensidhaltiges wässriges Mittel, welches Antischuppenpigmente, vorzugsweise Zink Pyrithion, enthält, eingebracht wird.

Ein besonderer Vorteil des erfindungsgemäßen Stabilisierungsgemischs ist dessen Eignung, in einem Kaltverfahren mit einem tensidischen wässrigen Mittel, welches Perlglanzsubstanzen enthält, vermischt zu werden, wobei ein stabilisiertes Perlglanzmittel erhalten wird. Damit ist insbesondere gemeint, dass die enthaltene Perlglanzsubstanz bei üblichen Lagerungsbedingungen nicht aggregiert und/oder abgeschieden wird, sodass keine sichtbare Veränderung oder Phasentrennung auftritt. Zur Herstellung eines stabilen tensidischen Perlglanzmittels wird durch die vorliegende Erfindung also ein äußerst einfaches Verfahren bereitgestellt. Nachteile eines weiteren Erwärmungsschritts und von dadurch bedingen Qualitätsschwankungen und -einbußen werden dadurch vermieden.

Mit "Kaltverfahren" ist erfindungsgemäß gemeint, dass die Vermischung mit dem tensidischen Perlglanzmittel bei einer Temperatur des Stabilisierungsgemischs von üblicherweise 15 bis 35 °C durchgeführt wird. Dies schließt also Temperaturen etwas oberhalb Raumtemperatur ein, bei denen die Verarbeitbarkeit (z.B. Pumpbarkeit und Abfüllbarkeit) des Mischansatzes vereinfacht wird. Es ist überraschend, dass eine noch höhere Temperatur, bspw. um das dispergierte hydrierte Ricinusöl zunächst zu schmelzen, nicht erforderlich ist.

Bei der erfindungsgemäßen Herstellung eines tensidhaltigen wässrigen Mittels mit Perlglanzeffekt ist das Mischungsverhältnis der Dispersion zum Reinigungsmittel bevorzugt etwa 1:5 bis 1:20 beträgt, bevorzugt etwa 1:5 bis 1:10, besonders bevorzugt etwa 1:9. Allgemein kann das Mischungsverhältnis so gewählt werden, dass man in dem hergestellten tensidhaltigen wässrigen Mittel eine Endkonzentration an hydriertem Ricinusöl von etwa 0,1 bis 1 Gew.-% erhält, bevorzugter etwa 0,2 bis 0,5 Gew.-%. Gegebenenfalls kann das Stabilisierungsgemisch dafür zuvor noch mit Wasser verdünnt werden.

Wird die erfindungsgemäße Dispersion bzw. des Stabilisierungsgemischs zur Stabilisierung von tensidhaltigen, wässrigen Mitteln vor der Vermischung mit dem Reinigungsmittel mit Wasser verdünnt, so beträgt das Mischungsverhältnis von erfindungsgemäßer Dispersion zu Verdünndungswasser vorzugsweise 1:9 bis 1:1, bevorzugt 1:5 bis 1:1.

Die nach der Verdünnung erhaltene wässrige Dispersion von hydriertem Ricinusöl zur Stabilisierung von Perlglanzwachse und/oder Perlglanzpigmente enthaltenden, wässrigen tensidischen Mitteln, enthält, bezogen auf ihr Gesamtgewicht, vorzugsweise:
(a) 1 bis 15 Gew.-% hydriertes Ricinusöl,
(b) 0,2 bis 10 Gew.-% eines oder mehrerer anionischer Tenside,
(c) 1 bis 12 Gew.-% eines oder mehrerer amphoterer Tenside, und
(d) 1 bis 12 Gew.-% eines oder mehrerer nichtionischer Tenside
(e) 70 bis 96 Gew.-% Wasser.

Die vorliegende Erfindung betrifft auch die Verwendung der erfindungsgemäßen Dispersion bzw. des Stabilisierungsgemischs zur Stabilisierung von tensidhaltigen, wässrigen Mitteln, insbesondere Shampoos oder Duschgelen, welche Perlglanzwachse und/oder Perlglanzpigmente enthalten.

Die vorliegende Erfindung betrifft weiterhin die Verwendung der erfindungsgemäßen Dispersion bzw. des Stabilisierungsgemischs zur Stabilisierung von tensidhaltigen, wässrigen Mitteln, insbesondere Shampoos oder Duschgelen, welche Antischuppenpigmente, insbesondere Zink Pyrithion enthalten.

In einer bevorzugten Ausführungsform der erfindungsgemäßen Verwendungen beträgt der Gewichtsanteil erfindungsgemäßen Dispersion bzw. des Stabilisierungsgemischs zur Stabilisierung von tensidhaltigen, wässrigen Mitteln am Gesamtgewicht des tensidhaltigen, wässrigen Mittels 0,5 bis 10 Gew.-%, vorzugsweise 1 bis 8 Gew.-% und insbesondere 1,5 bis 6 Gew.-%.

### Tabellarische Übersicht:

Im Folgenden sind bevorzugte erfindungsgemäße Stabilisierungsgemische aufgeführt. Die Angaben sind jeweils in Gew.-% und beziehen sich auf die Aktivstoffkonzentration.

| | Formel 1 | Formel 2 | Formel 3 | Formel 4 |
|---|---|---|---|---|
| Hydrogenated Castor Oil | 1 bis 25 | 2 bis 20 | 2,5 bis 15 | 5 bis 10 |
| Anionische(s) Tensid(e) | 2 bis 30 | 2 bis 15 | 2 bis 10 | 2,5 bis 5 |
| Amphotere(s) Tenside(e) | 2 bis 30 | 3 bis 20 | 5 bis 15 | 5 bis 15 |
| Nichtionische(s) Tensid(e) | 2 bis 30 | 3 bis 20 | 5 bis 15 | 5 bis 15 |
| Misc | add 100 | add 100 | add 100 | add 100 |

| | Formel 1a | Formel 2a | Formel 3a | Formel 4a |
|---|---|---|---|---|
| Hydrogenated Castor Oil, Schmelzpunkt 85-88°C | 1 bis 25 | 2 bis 20 | 2,5 bis 15 | 5 bis 10 |
| Anionische(s) Tensid(e): C₁₀-C₁₈-Alkylethersulfat | 2 bis 30 | 2 bis 15 | 2 bis 10 | 2,5 bis 5 |
| Amphotere(s) Tenside(e): Betain(e) und/oder Sultain(e) | 2 bis 30 | 3 bis 20 | 5 bis 15 | 5 bis 15 |
| Nichtionische(s) Tensid(e): | 2 bis 30 | 3 bis 20 | 5 bis 15 | 5 bis 15 |
| - Ethylenoxid-Anlagerungsprodukt an gesättigte lineare Fettalkohole und/oder Fettsäuren mit jeweils 2 bis 30 Mol Ethylenoxid pro Mol Fettalkohol und/oder Fettsäure, und/oder | | | | |
| - C₁₂-C₃₀-Fettsäureester von Anlagerungsprodukten von 1 bis 30 Mol Ethylenoxid an Glycerin, und/oder | | | | |
| - Anlagerungsprodukte von 5 bis 60 Mol Ethylenoxid an gehärtetes Ricinusöl | | | | |
| pH | 4 bis 6 | 4 bis 6 | 4 bis 6 | 4 bis 6 |
| Misc | add 100 | add 100 | add 100 | add 100 |

| | Formel 1b | Formel 2b | Formel 3b | Formel 4b |
|---|---|---|---|---|
| Hydrogenated Castor Oil, Schmelzpunkt 85-88°C | 1 bis 25 | 2 bis 20 | 2,5 bis 15 | 5 bis 10 |
| Anionische(s) Tensid(e): Alkali- oder Ammoniumsalz des Laurylethersulfats mit einem Ethoxylierungsgrad von 2 bis 4 Ethylenoxidgruppen | 2 bis 30 | 2 bis 15 | 2 bis 10 | 2,5 bis 5 |
| Amphotere(s) Tenside(e): Cocamidopropyl Hydroxysultaine (INCI) und/oder Cocamidopropyl Betaine (INCI) | 2 bis 30 | 3 bis 20 | 5 bis 15 | 5 bis 15 |
| Nichtionische(s) Tensid(e): Laureth-4 (INCI), PEG-7 Glycerol Cocoate (INCI) und/oder PEG-40 Hydrogenated Castor Oil (INCI) | 2 bis 30 | 3 bis 20 | 5 bis 15 | 5 bis 15 |
| pH | 4 bis 6 | 4 bis 6 | 4 bis 6 | 4 bis 6 |
| Misc | add 100 | add 100 | add 100 | add 100 |

Unter "Misc" sind erfindungsgemäß ist erfindungsgemäß im Wesentlichen Waser zu verstehen. Optional können ein Säure zur pH-Einstellung oder ein Konservierungsmittel enthalten sein. Bevorzugt ist fällt unter "Misc" kein kationisches Tensid.

### Beispiele

### 1. Stabilisierungsgemische

Es wurden die in der folgenden Tabelle 1 aufgeführten erfindungsgemäßen wässrigen Dispersionen von gehärtetem Ricinusöl hergestellt. Die Mengenangaben bedeuten, sofern nichts anderes angegeben ist, Gewichtsprozent.

Die Herstellung erfolgte, indem ein erstes Gemisch aus der Hälfte des jeweils verwendeten Wassers, dem anionischen und dem amphoteren Tensid, der Zitronensäure und des Natriumbenzoats, vorgelegt wurde, gelöst und auf 80°C erwärmt wurde. Anschließend wurde ein zweites Gemisch aus dem hydriertem Ricinusöl und dem nichtionischen Tensid, das zunächst bei z.B. 90-100 °C aufgeschmolzen worden war, zu dem ersten Gemisch gegeben und es wurde bei einer Ansatztemperatur von etwa 80 bis 90 °C vermischt und 15 min im Heißen homogenisiert. Dann wurde jeweils der restliche Teil des Wassers im nicht erwärmten Zustand zugegeben und das Gemisch wurde unter Rühren im Vakuum und Homogenisieren auf 35 °C abgekühlt.

Der pH-Wert der erhaltenen Stabilisierungsgemische lag jeweils im Bereich von 4,5 bis 5,5. Und die Viskosität bei unter 10000 mPas (Brookfield-Viskosimeter, 20°C, 60 s, Spindel 5, 20 UpM).

**Tab. 1**

| **INCI oder andere Bezeichnung** | **Tensidtyp** | **Beispiel 1** | **Beispiel 2** | **Beispiel 3** | **Beispiel 4** | **Beispiel 5** |
|---|---|---|---|---|---|---|
| Water | - | 44,25 | 41,75 | 39,25 | 51,80 | 46,75 |
| Sodium Benzoate | - | 0,50 | 0,50 | 0,50 | 0,50 | 0,50 |
| Citric Acid | - | 0,25 | 0,25 | 0,25 | 0,25 | 0,25 |
| Cocamidopropyl Hydroxysultaine 50% | Amphoter | 25,00 | 30,00 | - | - | - |
| Cocamidopropyl Betaine 40% | Amphoter | - | - | 30,00 | 15,00 | 30,00 |
| Sodium Laureth Sulfate 25 % | Anionisch | 15,00 | 10,00 | 10,00 | 15,00 | 10,00 |
| Hydrogenated Castor Oil, Schmelzpunkt 85-88°C | - | 5,00 | 10,00 | 5,00 | 10,00 | 5,00 |
| Laureth-4 | Nichtionisch | - | 7,50 | - | - | 7,50 |
| PEG-40 Hydrogenated Castor Oil | Nichtionisch | - | - | - | 7,50 | - |
| PEG-7 Glyceryl Cocoate | Nichtionisch | 10,00 | - | 15,00 | - | - |
| | | 100,00 | 100,00 | 100,00 | 100,00 | 100,00 |

Das Aussehen der Stabilisierungsgemische der Beispiele 1 bis 5 war jeweils milchig homogen.

### 2. Herstellung von stabilisierten Perlglanz-Shampoos

Die Stabilisierungsgemische, die in den Beispielen 1 bis 5 erhalten wurden, die eine Temperatur von 35 °C aufwiesen, wurden mit einem unten in Tabelle 2 angegebenen Basis-Shampoo, das Umgebungstemperatur aufwies, unter Rühren vermischt. Für das Vermischen wurden die Stabilisierungsgemische zunächst in den in Tabelle 2 angegebenen Verhältnissen verdünnt.

Zum Vergleich wurde das Basis-Shampoo einmal mit einer entsprechenden Menge Wasser vermischt und einmal durch Direktzugabe von hydriertem Ricinusöl in einem Heißverfahren. Das Mischungsverhältnis von Stabilisierungsgemisch bzw. Vergleichsgemisch zu Basis-Shampoo betrug jeweils 1:9.

Der pH-Wert der erhaltenen Stabilisierungsgemische lag jeweils im Bereich von 4,5 bis 5,5. Und die Viskosität bei 7000 bis 10000 mPas (Brookfield-Viskosimeter, 20°C, 60 s, Spindel 5, 20 UpM).

Die Stabilitäten der erhaltenen stabilisierten Shampoos wurden nach 5 Tagen Lagerung bei 45 °C und bei 50 °C visuell bewertet. Die Ergebnisse sind in Tabelle 3 dargestellt.

**Tab. 2 Basis-Shampoo und Mischung mit Stabilisierungsgemischen**

| | | **Keine Stabilis. (Wasserzugabe)** | **Stabilis. mit Hydrogen. Castor Oil (Heißverfahren)** | **Stab. mit Bsp. 1** | **Stab. mit Bsp. 2** | **Stab. mit Bsp. 3** | **Stab. mit Bsp. 4** | **Stab. mit Bsp. 5** |
|---|---|---|---|---|---|---|---|---|
| **INCI oder andere Bezeichnung** | | | | | | | | |
| **Basis-Shampoo** | | | | | | | | |
| Sodium Laureth Sulfate 70% | 14,50 | 14,50 | 14,50 | 14,50 | 14,50 | 14,50 | 14,50 | 14,50 |
| Water | 53,65 | 53,65 | 53,65 | 53,65 | 53,65 | 53,65 | 53,65 | 53,65 |
| Disodium Cocoamphodiacetate | 8,00 | 8,00 | 8,00 | 8,00 | 8,00 | 8,00 | 8,00 | 8,00 |
| PEG-120 Methyl Glucose Dioleate | 0,30 | 0,30 | 0,30 | 0,30 | 0,30 | 0,30 | 0,30 | 0,30 |
| Sodium Benzoate | 0,30 | 0,30 | 0,30 | 0,30 | 0,30 | 0,30 | 0,30 | 0,30 |
| CI 77891 (Titanium Oxide) & CI 77491 (Iron Oxide) % Mica | 0,30 | 0,30 | 0,30 | 0,30 | 0,30 | 0,30 | 0,30 | 0,30 |
| Parfum (Fragrance) | 0,50 | 0,50 | 0,50 | 0,50 | 0,50 | 0,50 | 0,50 | 0,50 |
| PEG-40 Hydrogenated Castor Oil | 0,50 | 0,50 | 0,50 | 0,50 | 0,50 | 0,50 | 0,50 | 0,50 |
| Water | 10,00 | 10,00 | 10,00 | 10,00 | 10,00 | 10,00 | 10,00 | 10,00 |
| Polyquaternium -10 | 0,40 | 0,40 | 0,40 | 0,40 | 0,40 | 0,40 | 0,40 | 0,40 |
| Citric Acid | 0,75 | 0,75 | 0,75 | 0,75 | 0,75 | 0,75 | 0,75 | 0,75 |
| Sodium Chloride | 0,80 | 0,80 | 0,80 | 0,80 | 0,80 | 0,80 | 0,80 | 0,80 |
| | | | | | | | | |

| **Zugabeansatz** | | | | | | | | |
|---|---|---|---|---|---|---|---|---|
| Water | | 10,00 | 9,00* | 6,00 | 8,00 | 6,00 | 8,00 | 6,00 |
| Hydrogenated Castor Oil | | | 0,20** | | | | | |
| PEG-7 Glyceryl Cocoate | | | 0,80** | | | | | |
| Bsp. 1 | | | | 4,00 | | | | |
| Bsp. 2 | | | | | 2,00 | | | |
| Bsp. 3 | | | | | | 4,00 | | |
| Bsp. 4 | | | | | | | 2,00 | |
| Bsp. 5 | | | | | | | | 4,00 |
| Gesamt | 90 | 100 | 100 | 100 | 100 | 100 | 100 | 100 |

| | | | | | | | | |
|---|---|---|---|---|---|---|---|---|
| *Im Fall der Stabilisierung mit Hydrogenated Castor Oil wurden zunächst die 9 Teile Wasser auf 80°C erwärmt ** Hydrogenated Castor Oil und PEG-7 Glyceryl Cocoate wurden bei 85 °C zusammen aufgeschmolzen und zu dem Wasser auf 85 erwärmten Wasser gegeben. | | | | | | | | |

**Tabelle 3 - Stabilitätstests**

| | **Lagertemperatur** | **Bewertung** |
|---|---|---|
| Keine Stabilisierung (Wasserzugabe) | 45 °C | vollständige Separation |
| | 50 °C | vollständige Separation |
| Stabilisierung ohne Hydrogenated Castor Oil (Heißverfahren) | 45 °C | deutliche Separation |
| | 50 °C | deutliche Separation |
| Stabilisierung mit Bsp. 1 | 45 °C | keine Veränderung |
| | 50 °C | leichte Separation |
| Stabilisierung mit Bsp. 2 | 45 °C | keine Veränderung |
| | 50 °C | keine Veränderung |
| Stabilisierung mit Bsp. 3 | 45 °C | keine Veränderung |
| | 50 °C | keine Veränderung |
| Stabilisierung mit Bsp. 4 | 45 °C | keine Veränderung |
| | 50 °C | keine Veränderung |
| Stabilisierung mit Bsp. 5 | 45 °C | keine Veränderung |
| | 50 °C | leichte Separation |

Es zeigte sich demnach eine klare Verbesserung der Stabilität bei erhöhten Temperaturen, wenn die erfindungsgemäßen Stabilisierungsgemische kalt in ein Basis-Shampoo eingemischt wurden.

## Patentansprüche

1. Wässrige Dispersion von hydriertem Ricinusöl zur Stabilisierung von Perlglanzwachse und/oder Perlglanzpigmente enthaltenden, wässrigen tensidischen Mitteln, welche, bezogen auf ihr Gesamtgewicht, enthält:
(a) 1 bis 25 Gew.-% hydriertes Ricinusöl,
(b) 2 bis 30 Gew.-% eines oder mehrerer anionischer Tenside,
(c) 2 bis 30 Gew.-% eines oder mehrerer amphoterer Tenside, und
(d) 2 bis 30 Gew.-% eines oder mehrerer nichtionischer Tenside.

2. Dispersion nach Anspruch 1, welche, bezogen auf ihr Gesamtgewicht, enthält:
(a) 2,5 bis 15 Gew.-% hydriertes Ricinusöl,
(b) 2,5 bis 10 Gew.-% eines oder mehrerer anionischer Tenside,
(c) 5 bis 15 Gew.-% eines oder mehrerer amphoterer Tenside, und
(d) 5 bis 15 Gew.-% eines oder mehrerer nichtionischer Tenside.

3. Dispersion nach Anspruch 1 oder 2, wobei die Dispersion kein kationisches Tensid enthält.

4. Dispersion nach einem der vorhergehenden Ansprüche,
wobei als anionisches Tensid (b) ein Alkali- oder Ammoniumsalz des Laurylethersulfats mit einem Ethoxylierungsgrad von 2 bis 4 Ethylenoxidgruppen oder ein Gemisch davon enthalten ist.

5. Dispersion nach einem der vorhergehenden Ansprüche,
wobei als das amphotere Tensid (c) ein oder mehrere Betaine enthalten sind, bevorzugt Cocamidopropyl Hydroxysultaine (INCI) und/oder Cocamidopropyl Betaine (INCI).

6. Dispersion nach einem der vorhergehenden Ansprüche,
wobei das nichtionische Tensid (d) aus
- Ethylenoxid-Anlagerungsprodukten an gesättigte lineare Fettalkohole und/oder Fettsäuren mit jeweils 2 bis 30 Mol Ethylenoxid pro Mol Fettalkohol und/oder Fettsäure,
- C₁₂-C₃₀-Fettsäureestern von Anlagerungsprodukten von 1 bis 30 Mol Ethylenoxid an Glycerin,
- Anlagerungsprodukten von 5 bis 60 Mol Ethylenoxid an gehärtetes Ricinusöl und
- Gemischen davon
ausgewählt ist.

7. Dispersion nach einem der vorhergehenden Ansprüche, wobei das nichtionische Tensid (d) aus Laureth-4 (INCI), PEG-7 Glycerol Cocoate (INCI), PEG-40 Hydrogenated Castor Oil (INCI) und Gemischen davon ausgewählt ist.

8. Verfahren zur Herstellung eines stabilisierten tensidhaltigen wässrigen Mittels mit Perlglanzeffekt, bei dem eine Dispersion nach einem der Ansprüche 1 bis 7 in einem Kaltverfahren in ein tensidhaltiges, wässriges Mittel, welches Perlglanzwachse und/oder Perlglanzpigmente enthält, eingebracht wird.

9. Verfahren nach Anspruch 8, wobei die Menge des eingebrachten hydrierten Ricinusöls in dem stabilisierten tensidhaltigen wässrigen Mittel 0,1 bis 1 Gew.-% beträgt, bevorzugt 0,2 bis 0,5 Gew.-%.

10. Verwendung einer Dispersion nach einem der Ansprüche 1 bis 8 zur Stabilisierung von tensidhaltigen Mitteln, insbesondere kosmetischen Reinigungsmitteln, welche Perlglanzwachse und/oder Perlglanzpigmente und/oder Antischuppenpigmente enthalten.

## Claims

1. An aqueous dispersion of hydrogenated castor oil for stabilizing aqueous surfactant agents containing pearlescent waxes and/or pearlescent pigments, which dispersion contains, based on its total weight:
(a) 1 to 25 wt.% of hydrogenated castor oil,
(b) 2 to 30 wt.% of one or more anionic surfactants,
(c) 2 to 30 wt.% of one or more amphoteric surfactants, and
(d) 2 to 30 wt.% of one or more non-ionic surfactants.

2. The dispersion according to claim 1 which contains, based on its total weight:
(a) 2.5 to 15 wt.% of hydrogenated castor oil,
(b) 2.5 to 10 wt.% of one or more anionic surfactants,
(c) 5 to 15 wt.% of one or more amphoteric surfactants, and
(d) 5 to 15 wt.% of one or more non-ionic surfactants.

3. The dispersion according to claim 1 or 2, wherein the dispersion does not contain a cationic surfactant.

4. The dispersion according to one of the preceding claims, wherein an alkali or ammonium salt of lauryl ether sulfate that has a degree of ethoxylation of from 2 to 4 ethylene oxide groups or a mixture thereof is contained as the anionic surfactant (b).

5. The dispersion according to one of the preceding claims, wherein one or more betaines are contained as the amphoteric surfactant (c), preferably cocamidopropyl hydroxysultaines (INCI) and/or cocamidopropyl betaines (INCI).

6. The dispersion according to one of the preceding claims, wherein the non-ionic surfactant (d) is selected from
- ethylene oxide addition products of saturated linear fatty alcohols and/or fatty acids, each having 2 to 30 mol of ethylene oxide per mol of fatty alcohol and/or fatty acid,
- C₁₂-C₃₀ fatty acid esters of addition products of 1 to 30 mol of ethylene oxide to glycerol, addition products of 5 to 60 mol of ethylene oxide to hardened castor oil, and
- mixtures thereof.

7. The dispersion according to one of the preceding claims, wherein the non-ionic surfactant (d) is selected from laureth-4 (INCI), PEG-7 glycerol cocoate (INCI), PEG-40 hydrogenated castor oil (INCI) and mixtures thereof.

8. A method for producing a stabilized surfactant-containing aqueous agent which has a pearlescent effect, in which method a dispersion according to one of claims 1 to 7 is introduced by means of a cold process into a surfactant-containing aqueous agent which contains pearlescent waxes and/or pearlescent pigments.

9. The method according to claim 8, wherein the amount of hydrogenated castor oil introduced into the stabilized surfactant-containing aqueous agent is 0.1 to 1 wt.%, preferably 0.2 to 0.5% wt.%.

10. The use of a dispersion according to one of claims 1 to 8 for stabilizing surfactant-containing agents, in particular cosmetic cleaning agents, which contain pearlescent waxes and/or pearlescent pigments and/or anti-dandruff pigments.

## Revendications

1. Dispersion aqueuse d'huile de ricin hydrogénée destinée à la stabilisation d'agents tensioactifs aqueux contenant des cires à éclat nacré et/ou des pigments à éclat nacré, laquelle dispersion contient, par rapport à son poids total :
(a) 1 à 25 % en poids d'huile de ricin hydrogénée,
(b) 2 à 30 % en poids d'un ou de plusieurs tensioactifs anioniques,
(c) 2 à 30 % en poids d'un ou de plusieurs tensioactifs amphotères, et
(d) 2 à 30 % en poids d'un ou de plusieurs tensioactifs non ioniques.

2. Dispersion selon la revendication 1, laquelle dispersion contient, par rapport à son poids total :
(a) 2,5 à 15 % en poids d'huile de ricin hydrogénée,
(b) 2,5 à 10 % en poids d'un ou de plusieurs agents tensioactifs anioniques,
(c) 5 à 15 % en poids d'un ou de plusieurs tensioactifs amphotères, et
(d) 5 à 15 % en poids d'un ou de plusieurs tensioactifs non ioniques.

3. Dispersion selon la revendication 1 ou 2, la dispersion ne contenant aucun tensioactif cationique.

4. Dispersion selon l'une des revendications précédentes, dans laquelle un sel alcalin ou d'ammonium de lauryléthersulfate ayant un degré d'éthoxylation de 2 à 4 groupes oxyde d'éthylène ou un mélange de ceux-ci est présent comme agent tensioactif anionique (b).

5. Dispersion selon l'une des revendications précédentes, dans laquelle une ou plusieurs bétaines, de préférence des cocamidopropyl hydroxysultaines (INCI) et/ou des cocamidopropyl bétaines (INCI) sont présentes comme tensioactif amphotère (c).

6. Dispersion selon l'une des revendications précédentes, dans laquelle le tensioactif non ionique (d) est choisi parmi les
- produits d'addition d'oxyde d'éthylène à des alcools gras linéaires saturés et/ou à les acides gras, contenant respectivement 2 à 30 moles d'oxyde d'éthylène par mole d'alcool gras et/ou d'acide gras,
- esters d'acides gras en C₁₂-C₃₀ de produits d'addition de 1 à 30 moles d'oxyde d'éthylène au glycérol, de 5 à 60 moles d'oxyde d'éthylène à l'huile de ricin durcie, et
- des mélanges de ceux-ci.

7. Dispersion selon l'une des revendications précédentes, dans laquelle le tensioactif non ionique (d) est choisi parmi le laureth-4 (INCI), le cocoate de glycérol PEG-7 (INCI), l'huile de ricin hydrogénée PEG-40 (INCI) et des mélanges de ceux-ci.

8. Procédé de préparation d'une composition aqueuse stabilisée contenant un tensioactif ayant un effet à éclat nacré, dans lequel une dispersion selon l'une des revendications 1 à 7 est introduite dans un procédé à froid dans une composition aqueuse contenant un tensioactif qui contient des cires à éclat nacré et/ou des pigments à éclat nacré.

9. Procédé selon la revendication 8, dans lequel la quantité d'huile de ricin hydrogénée introduite dans la composition aqueuse stabilisée contenant un tensioactif est de 0,1 à 1 % en poids, de préférence de 0,2 à 0,5 % en poids.

10. Utilisation d'une dispersion selon l'une des revendications 1 à 8 pour stabiliser des composition contenant des tensioactifs, en particulier des produits de nettoyage cosmétiques qui contiennent des cires à éclat nacré et/ou des pigments à éclat nacré et/ou des pigments antipelliculaires.
